# EUROPEAN PATENT APPLICATION

(11) **EP 4 134 448 A1**
(43) Date of publication of application: **15.02.2023**
(21) Application number: 21194901.1
(22) Date of filing: 03.09.2021
(51) Int. Cl.: C12Q 1/6883

(54) **METHOD FOR GENETIC SELECTION OF SHEEP WITH INCREASED RESISTANCE TO FOOTROT**

(30) Priority: 13.08.2021 PT 2021117397
(71) Applicant: ACOS - Associação de Agricultores do Sul, 7800-453 Beja (PT); Cebal - Centro De Biotecnologia Agrícola E Agro-Alimentar Do Alentejo, 7800-309 Beja (PT); Universidade De Évora, 7004-516 Évora (PT); INIAV - Instituto Nacional de Investigação Agrária E Veterinária, 2780-157 Oeiras (PT)
(72) Inventor: PEREIRA MATOS, Claudino António, 7800-453 BEJA (PT); COSTA DO AMARAL RAMOS, António Markos, 7800-309 BEJA (PT); USIÉ CHIMENOS, Ana Isabel, 7800-309 BEJA (PT); BRANCO GASPAR, Daniel Filipe, 7800-309 BEJA (PT); GODINHO VIEIRA MONTEIRO, Maria Helena, 7800-453 BEJA (PT); FIALHO LEÃO, Célia Cristina, 2780-157 OEIRAS (PT); PIMENTEL CAROLINO, Renato Nuno, 2005-048 VALE DE SANTARÉM (PT); DA SILVA BRANCO, Sandra Maria, 7002-554 ÉVORA (PT); NETO PADRE, Ludovina, 7002-554 ÉVORA (PT); VARELA BETTENCOURT HENRIQUES, Elisa Maria, 7002-554 ÉVORA (PT); DE SOUSA HENRIQUES, Pedro Damião, 7000-803 ÉVORA (PT)
(74) Representative: Neves, Ana

(57) **Abstract**

The present patent application discloses a method for the selection of sheep based on their footrot resistance profile. Said footrot resistance profile is determined based on a group of Single Nucleotide Polymorphisms (SNPs) associated with lower scores for footrot.

## Description

### Technical field

The present patent application relates to a method for the genetic selection of sheep based on their resistance to footrot.

### Background art

Footrot is one of the main causes of lameness in sheep and represents a major animal welfare problem due to the painful nature of the lesions. This disease is caused by infection with *Dichelobacter nodosus (D. nodosus),* causing painful inflammation, necrotizing lesions of the interdigital skin, characteristic odor, and hoof detachment. Footrot is a multifactorial disease and its severity depends on the breed of sheep, farm management, environmental factors, the presence of co-infecting bacteria, and the virulence of the *D. nodosus* strains involved. In addition, the bacterium *Fusobacterium necrophorum (F. necrophorum)* has also been suggested as a secondary pathogen in the development of the disease and probably in increasing its severity. The synergistic relationship between *F. necrophorum* and D. *nodosus* is not yet fully explained, but it appears that their combined action is involved in the development and severity of wheal.

In sheep, there is variation in the individual response of each animal to the footrot. Under the same environmental conditions, for example, on the same farm, some animals are severely affected by the disease, while others show no symptoms and seem unaffected. This clearly indicates the possibility of genetic control of footrot, in particular the severity with which each animal is affected. To date, no genetic markers for the footrot have been identified in Portuguese sheep breeds.

### Summary

The present patent application relates to a method for the selection of sheep based on footrot resistance genotypes.

The method for sheep selection based on their footrot resistance genotype comprises the following steps:
Preparation of a sheep biological sample and extraction of DNA from said sample;
Genotyping the DNA sample for at least one of the following Single Nucleotide Polymorphisms (SNPs),

| Chromosome | Position | Exonic alteration |
|---|---|---|
| NC_040254.1 | 14627723 | - |
| NC_040254.1 | 78539086 | - |
| NC_040254.1 | 237741074 | synonym |
| NC_040263.1 | 62286092 | - |
| NC_040266.1 | 71702732 | - |
| NC_040269.1 | 21731867 | synonym |
| NC_040276.1 | 24122307 | - |
| NC_040276.1 | 27418667 | - |
| NC_040259.1 | 13386681 | - |
| NC_040278.1 | 50565062 | - |

Determining sheep's resistance to footrot based on the presence of at least one of said SNPs significantly associated with a lower score for footrot.

In one embodiment, the sample analyzed is preferably a blood sample.

In another embodiment, the above-mentioned Single Nucleotide Polymorphyms (SNPs) are used as biomarkers for footrot in sheep.

### Detailed Description

The present patent application discloses a method for the selection of sheep based on their resistance to footrot. Said resistance to footrot is determined based on a group of Single Nucleotide Polymorphisms (SNPs) disclosed herein which are associated with lower scores of footrot in sheep.

The technology disclosed herein is based on the sequencing of genomes of animals of the breeds that will later be used in genome-wide association studies. This step of genome sequencing is essential to characterize in great detail all the type of variation present in the animals studied. The next step involves the selection of a set of SNPs to be genotyped in a larger number of animals, which in this case was 1500.All genotyped animals were also characterized from the phenotypic point of view, namely in the determination of the lameness score observed in each animal's paw, in two distinct visits made on the same farm, and spaced temporally for a period of approximately 3 months.

After all animals were genotyped, a genome-wide association study was performed, where the statistical significance of the association observed between each genotyped SNP and the phenotypic value of the wheal score was determined. This significance was determined after a correction for multiple testing, an essential step in this type of analysis.

For each of the significant SNPs the observed mean for each of the genotypes is determined. The difference observed is the potential phenotypic gain that can be achieved if animals carrying the genotypes associated with a lower litter score are genetically selected.

The 10 SNPs identified are described in Table 1.

| Chromosome | Position | P-Value | Gene ID | Genomic Localization | Exonic alteration |
|---|---|---|---|---|---|
| NC_040254.1 | 14627723 | 0.0003312 | gene-DAB2IP | intron | - |
| NC_040254.1 | 78539086 | 0.0005151 | gene-NRXN1 | intron | - |
| NC_040254.1 | 237741074 | 0.000126 | gene-CELSR1 | exon | synonym |
| NC_040263.1 | 62286092 | 0.0005151 | gene-ASTN1 | intron | - |
| NC_040266.1 | 71702732 | 0.0005477 | gene-LDLRAD3 | intron | - |
| NC_040269.1 | 21731867 | 0.0005151 | gene-ADAMTSL3 | exon | synonym |
| NC_040276.1 | 24122307 | 0.0001633 | gene-CTNNA3 | intron | - |
| NC_040276.1 | 27418667 | 0.0003593 | gene-LRRC20 | UTR3 | - |
| NC_040259.1 | 13386681 | 0.0002215 | | intergenic | - |
| NC_040278.1 | 50565062 | 0.000126 | | intergenic | - |

For the 11 SNPs identified, the observed mean values are shown in Table 2.

| | | | | | |
|---|---|---|---|---|---|
| NC_040254.1 | NC_040254.1#14627723 | GENO | A/A | A/G | G/G |
| NC_040254.1 | NC_040254.1#14627723 | COUNTS | 9 | 121 | 1229 |
| NC_040254.1 | NC_040254.1#14627723 | FREQ | 0.006623 | 0.08904 | 0.9043 |
| NC_040254.1 | NC_040254.1#14627723 | MEAN | 3.961 | 2.444 | 1.491 |
| NC_040254.1 | NC_040254.1#14627723 | SD | 2.539 | 2.854 | 2.215 |
| | | | | | |
| NC_040254.1 | NC_040254.1#78539086 | GENO | T/T | T/C | C/C |
| NC_040254.1 | NC_040254.1#78539086 | COUNTS | 9 | 181 | 1247 |
| NC_040254.1 | NC_040254.1#78539086 | FREQ | 0.006263 | 0.126 | 0.8678 |
| NC_040254.1 | NC_040254.1#78539086 | MEAN | -0.1364 | 0.8893 | 1.733 |
| NC_040254.1 | NC_040254.1#78539086 | SD | 0.4949 | 2.068 | 2.315 |
| | | | | | |
| NC_040254.1 | NC_040254.1#237741074 | GENO | T/T | T/C | C/C |
| NC_040254.1 | NC_040254.1#237741074 | COUNTS | 4 | 149 | 1268 |
| NC_040254.1 | NC_040254.1#237741074 | FREQ | 0.002815 | 0.1049 | 0.8923 |
| NC_040254.1 | NC_040254.1#237741074 | MEAN | 2.142 | 2.615 | 1.486 |
| NC_040254.1 | NC_040254.1#237741074 | SD | 2.307 | 2.919 | 2.174 |
| | | | | | |
| NC_040259.1 | NC_040259.1#13386681 | GENO | A/A | A/G | G/G |
| NC_040259.1 | NC_040259.1#13386681 | COUNTS | 5 | 99 | 881 |
| NC_040259.1 | NC_040259.1#13386681 | FREQ | 0.005076 | 0.1005 | 0.8944 |
| NC_040259.1 | NC_040259.1#13386681 | MEAN | 0.7679 | 2.803 | 1.353 |
| NC_040259.1 | NC_040259.1#13386681 | SD | 1.22 | 2.94 | 2.054 |
| | | | | | |
| NC_040263.1 | NC_040263.1#62286092 | GENO | G/G | G/A | A/A |
| NC_040263.1 | NC_040263.1#62286092 | COUNTS | 19 | 173 | 1153 |
| NC_040263.1 | NC_040263.1#62286092 | FREQ | 0.01413 | 0.1286 | 0.8572 |
| NC_040263.1 | NC_040263.1#62286092 | MEAN | 2.869 | 2.292 | 1.457 |
| NC_040263.1 | NC_040263.1#62286092 | SD | 3.486 | 2.608 | 2.162 |
| | | | | | |
| NC_040266.1 | NC_040266.1#71702732 | GENO | C/C | C/T | T/T |
| NC_040266.1 | NC_040266.1#71702732 | COUNTS | 160 | 631 | 638 |
| NC_040266.1 | NC_040266.1#71702732 | FREQ | 0.112 | 0.4416 | 0.4465 |
| NC_040266.1 | NC_040266.1#71702732 | MEAN | 1.147 | 1.371 | 1.954 |
| NC_040266.1 | NC_040266.1#71702732 | SD | 1.903 | 2.157 | 2.449 |
| | | | | | |
| NC_040269.1 | NC_040269.1#21731867 | GENO | A/A | A/G | G/G |
| NC_040269.1 | NC_040269.1#21731867 | COUNTS | 104 | 537 | 776 |
| NC_040269.1 | NC_040269.1#21731867 | FREQ | 0.07339 | 0.379 | 0.5476 |
| NC_040269.1 | NC_040269.1#21731867 | MEAN | 2.441 | 1.794 | 1.355 |
| NC_040269.1 | NC_040269.1#21731867 | SD | 2.877 | 2.391 | 2.077 |
| | | | | | |
| NC_040276.1 | NC_040276.1#24122307 | GENO | A/A | A/C | C/C |
| NC_040276.1 | NC_040276.1#24122307 | COUNTS | 28 | 312 | 1076 |
| NC_040276.1 | NC_040276.1#24122307 | FREQ | 0.01977 | 0.2203 | 0.7599 |
| NC_040276.1 | NC_040276.1#24122307 | MEAN | 2.356 | 2.182 | 1.403 |
| NC_040276.1 | NC_040276.1#24122307 | SD | 2.581 | 2.655 | 2.127 |
| | | | | | |
| NC_040276.1 | NC_040276.1#27418667 | GENO | T/T | T/C | C/C |
| NC_040276.1 | NC_040276.1#27418667 | COUNTS | 75 | 439 | 876 |
| NC_040276.1 | NC_040276.1#27418667 | FREQ | 0.05396 | 0.3158 | 0.6302 |
| NC_040276.1 | NC_040276.1#27418667 | MEAN | 2.416 | 1.914 | 1.359 |
| NC_040276.1 | NC_040276.1#27418667 | SD | 2.761 | 2.503 | 2.078 |
| | | | | | |
| NC_040278.1 | NC_040278.1#50565062 | GENO | G/G | G/A | A/A |
| NC_040278.1 | NC_040278.1#50565062 | COUNTS | 22 | 210 | 1198 |
| NC_040278.1 | NC_040278.1#50565062 | FREQ | 0.01538 | 0.1469 | 0.8378 |
| NC_040278.1 | NC_040278.1#50565062 | MEAN | 2.675 | 2.336 | 1.454 |
| NC_040278.1 | NC_040278.1#50565062 | SD | 2.368 | 2.612 | 2.192 |

The method of the present patent application involves several steps. Initially, it is necessary to collect a sample of biological material, such as a blood sample, for DNA extraction. Next, the DNA sample is genotyped for the SNPs of interest, and the genotypes obtained for each SNP are evaluated. The decision to select the animal is based on the presence of the genotype(s) significantly associated with a lower score for footrot, i.e. the goal is to select animals with higher resistance to footrot.

The method for sheep selection based on footrot resistance genotypes comprises the following steps:
Preparation of a sheep biological sample and extraction of DNA from said sample;
Genotyping the DNA sample for at least one of the following Single Nucleotide Polymorphisms (SNPs),

| Chromosome | Position | Exonic alteration |
|---|---|---|
| NC_040254.1 | 14627723 | - |
| NC_040254.1 | 78539086 | - |
| NC_040254.1 | 237741074 | synonym |
| NC_040263.1 | 62286092 | - |
| NC_040266.1 | 71702732 | - |
| NC_040269.1 | 21731867 | synonym |
| NC_040276.1 | 24122307 | - |
| NC_040276.1 | 27418667 | - |
| NC_040259.1 | 13386681 | - |
| NC_040278.1 | 50565062 | - |

Determining sheep's resistance to footrot based on the presence of at least one of said SNPs significantly associated with a lower score for footrot.

In one embodiment, the sample analyzed is preferably a blood sample.

## Claims

1. Method for sheep selection based on footrot resistance genotypes comprising the following steps:
- Preparation of a sheep biological sample and extraction of DNA from said sample;
- Genotyping the DNA sample for at least one of the following Single Nucleotide Polymorphisms (SNPs),
| Chromosome | Position | Exonic alteration |
|---|---|---|
| NC_040254.1 | 14627723 | - |
| NC_040254.1 | 78539086 | - |
| NC_040254.1 | 237741074 | synonym |
| NC_040263.1 | 62286092 | - |
| NC_040266.1 | 71702732 | - |
| NC_040269.1 | 21731867 | synonym |
| NC_040276.1 | 24122307 | - |
| NC_040276.1 | 27418667 | - |
| NC_040259.1 | 13386681 | - |
| NC_040278.1 | 50565062 | - |
- Determining sheep's resistance to footrot based on the presence of the at least one of said SNPs significantly associated with a lower score for footrot.

2. Single Nucleotide Polymorphyms (SNPs) as defined in claim 1 for use as biomarkers of footrot resistance in sheep.
